(19) Europäisches Patentamt
European Patent Office
Office européen des brevets

(11) Publication number: **0 409 527 A2**

# (12) EUROPEAN PATENT APPLICATION

(21) Application number: **90307746.9**

(51) Int. Cl.[5]: **C07F 15/00**, A61K 31/28

(22) Date of filing: **16.07.90**

(30) Priority: **18.07.89 US 381441**

(43) Date of publication of application:
**23.01.91 Bulletin 91/04**

(84) Designated Contracting States:
**AT BE CH DE DK ES FR GB GR IT LI LU NL SE**

(71) Applicant: **ENGELHARD CORPORATION**
**Menlo Park, CN 40**
**Edison New Jersey 08818(US)**

(72) Inventor: **Hollis, Leslie Steven**
**33 Guilford Lane**
**Mercerville, New Jersey 08619(US)**
Inventor: **Stern, Eric W.**
**234 Oak Tree Road**
**Mountainside, New Jersey 07092(US)**

(74) Representative: **Fisher, Adrian John et al**
**CARPMAELS & RANSFORD 43 Bloomsbury Squareare**
**London WC1A 2RA(GB)**

## (54) Cis-diamineplatinum complexes with methanediphosphonate and substituted methanediphosphonate ligands as antitumor agents.

(57) Novel cis-diamineplatinum (II) complexes contain methanediphosphonate ligands, $[(O_3P)_2C(R^1)(R^2]^{4-}$ wherein $R^1$ and $R^2$ represent certain substituents that can be used as points of attachment to a molecule of interest, e.g. an antibody, hormone, growth factor, glycolipid, oligosaccharide, amino acid, peptide, organic dye, DNA intercalation reagent, steroid or cytotoxic drug. The complexes and conjugates derived therefrom may be used to combat malignant tumor cells in animals.

EP 0 409 527 A2

# CIS-DIAMINEPLATINUM COMPLEXES WITH METHANEDIPHOSPHONATE AND SUBSTITUTED METHANEDIPHOSPHONATE LIGANDS AS ANTITUMOR AGENTS.

## Field of the Invention

The present invention relates to certain cis-diamineplatinum complexes and conjugates derived therefrom, to processes for the production of such complexes and conjugates, and to the use of such complexes and conjugates for combatting malignant tumors in animals.

## Background of the Invention

While cisplatin has proven effective as an antitumor agent in treating a variety of forms of cancer, such as testicular and ovarian carcinomas, a substantial effort has been made in new drug development programs to improve the therapeutic properties of platinum-based antitumor agents. Major objectives of research in this area include the development of new agents that have an improved spectrum of activity and are more effective and less toxic than cisplatin. To date, these efforts have produced a number of second generation analogs that are less toxic than cisplatin. See, for example, Carter, S. K., in "Platinum Coordination Compounds in Cancer Chemotherapy", Hacker, M.P.; Douple, E.B.; Krakoff, I.H., Eds.; Martinus Nijhoff: Boston, 1984, p. 259 and Dabrowiak, J.C.; Bradner, W.T., in "Progress in Medicinal Chemistry"; Ellis, G.P.; West, G.B., Eds.; Elsevier: Amsterdam, 1987; Vol. 24, p. 129. However, relatively little progress has been made toward developing agents that display a spectrum of activity that is significantly different from that of cisplatin.

One approach to enhance the activity and selectivity of platinum-based antitumor agents is to link platinum complexes to molecules that can serve as site-specific delivery agents. A large number of molecules can be considered for this purpose. For example, antibodies (Ab's), which have the ability to bind to cellular markers that are expressed on the surface of cancer cells (see "Monoclonal Antibodies in Cancer", J.A. Roth, Ed., Futura Pub. 1986), represent one class of molecules that can be used as delivery vehicles for platinum antitumor agents. By developing a platinum-Ab immunoconjugate, it may be possible to selectively deliver platinum complexes to cancerous cell populations, thereby reducing general cytotoxicity and enhancing drug concentration at the sites of interest. In addition, platinum complexes can be attached to a number of other biomolecules that can serve as delivery agents. A variety of hormones, growth factors, proteins, oligosaccharides, glycolipids, steroids and organic dyes are known to bind to specific cellular receptors or tissues and these agents also could be exploited for this purpose. Furthermore, platinum complexes can be linked to known cytotoxic agents, such as organic antitumor drugs, in an attempt to develop a conjugate that possesses enhanced potency and/or a different spectrum of activity.

Functionalized cis-diamineplatinum diphosphonate complexes represent one class of linkable platinum antitumor agents. These dimeric platinum complexes contain a methanediphosphonate (MDP) ligand with a functional group or groups attached to the central carbon (C1) of the diphosphonate ligand. Suitable functional groups are chosen such that the platinum-diphosphonate complexes can be coupled to the molecule of interest.

Two reports of compounds related to this series have appeared in print. In 1979, two dimeric platinum diphosphonate complexes were prepared and screened for antitumor activity (Nepras, M.J., Masters Thesis, Univ. of South Florida, Tampa, FL 1979). Both of these compounds, $[Pt_2(en)_2(MDP)]$ and $[Pt_2(en)_2(HEDP)]$ (where en = ethylenediamine and HEDP = 1-hydroxyethane-1,1-diphosphonate) were found to be inactive against the L1210 murine tumor system. In 1988, the synthesis and structure of $[Pt_2(cis\text{-}dach)_2(MDP)]$, where dach = 1,2-diaminocyclohexane, was reported by R. Bau et al (J. Am. Chem. Soc. 1988 , 110 , 7546), without mention of its potential utility as an antitumor agent.

The compounds described in this invention show advantage by displaying good activity against murine tumor systems. In addition, these agents can be functionalized and linked to a variety of molecules for the purpose of altering their toxicological and biodistribution properties.

Alternate methods of linking platinum complexes to molecules of biological interest have been described in one U.S. Patent (U.S. patent No 4,793,986) and two European patent applications (EPO No. 167,310 and EPO No. 169,645.

## Summary of the Invention

The present invention is directed to a series of platinum complexes that can be linked to various molecules for targeting purposes. This class of compounds is based on cis-diamineplatinum(II) complexes containing methanediphosphonate ligands, $[(O_3P)_2C(R^1)(R^2)]^{4-}$, wherein $R^1$ and $R^2$ represent a series of substituents that can be used as points of attachment to the molecules of interest. While these platinum compounds can be linked to various agents, they also have excellent activity in the absence of conjugation. In addition, they have good water solubility and excellent stability, particularly toward the reagents that are used in the linking reactions.

## Detailed Description of the Preferred Embodiment

The class of platinum complexes that are the subject of this invention may be described by the following general structural formula (I):

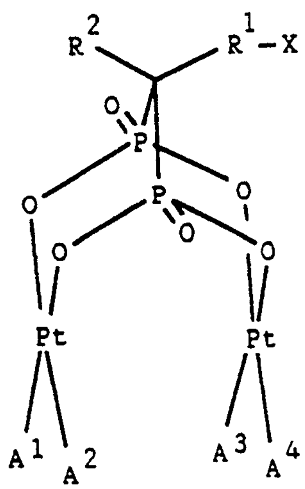

I

In this structure, the A ligands may be the same or different and are selected from a monodentate amine such as a ammonia, a primary alkylamine ($R^3NH_2$) or a secondary alkylamine ($R^3{}_2NH$) wherein $R^3$ is $C_1$-$C_6$ linear, cylcic or branched alkyl group. $A^1$ and $A^2$ or $A^3$ and $A^4$ may represent, when taken together on the same Pt, a bidentate diamine, such as a $C_2$ to $C_4$ alkyl such as ethylenediamine, or a $C_4$ to $C_8$ cycloalkyl diamine such as 1,2-diaminocyclohexane or 1,1-bisamino-methylcyclohexane. $R^1$ and $R^2$, on the methanediphosphonate ligand, may be the same or different, and are selected from H, alkoxy ($C_1$- $C_6$), linear or branched alkyl ($C_1$-$C_6$), linear or branched hydroxyalkyl ($C_1$-$C_6$), linear or branched alkyl ether ($C_1$-$C_6$), cycloalkyl ($C_4$-$C_8$), or phenyl. $R^1$ can also equal X as described herein. When $R^1$ is other than X, $R^1$ may be substituted with functional groups (X) such as amine, hydroxyl, halo, carboxylic acid, carboxylic acid halide, carboxylic acid anhydride, carboxylic acid hydrazide, ester, amide, aldehyde, acetal, hemiacetal, ketone, ketal, -O-acylurea, sulfonic acid ester, such as tosyl, mesyl or brosyl, hydrazine, $C_1$-$C_6$ linear or branched alkenyl in which the alkenyl group may contain any of the functional groups (X) as listed herein; phenyl, in which the phenyl group may contain any of the functional groups (X) listed herein. The functional groups (X) also can be attached directly to the central carbon of the methane-diphosphonate ligand. In this case, $R^1$ = X and X may be any of the functional groups listed herein. If the resulting complex is an anion, as in the case of a deprotonated carboxylic acid group, then the complex can be produced as a group IA salt ($Li^+$, $Na^+$, $K^+$, etc.), or an ammonium salt ($NH_4{}^+$ or $NR_4{}^+$, wherein R may be $C_1$-$C_6$ linear or branched alkyl substituent, etc.).

Not included within the scope of this invention are compounds wherein $A^1$ and $A^2$ and $A^3$ and $A^4$ are ethylene diamine and $R^1$ and $R^2$ are both H and $R^1$ and $R^2$ are $CH_3$ and OH, respectively. Also not included are compounds wherein $A^1$ and $A^2$ and $A^3$ and $A^4$ are cis-diaminocyclohexane and $R^1$ and $R^2$ are both H.

A number of approaches may be taken to prepare conjugates between the platinum-diphosphonate (Pt-DP) complexes described herein and the chosen molecule of interest (M). A few of these methods, which involve attaching a Pt-DP complex to a suitable linking group (Y), such as alcohols, amines, aldehydes, or carboxylic acids, on the target molecule M, are outlined below. The linkage (X′) in the resulting conjugate may be designed either to hydrolyze and release platinum, or to resist hydrolysis under physiological conditions. The preparation of Pt-DP based conjugates may be described by the following reaction sequence.

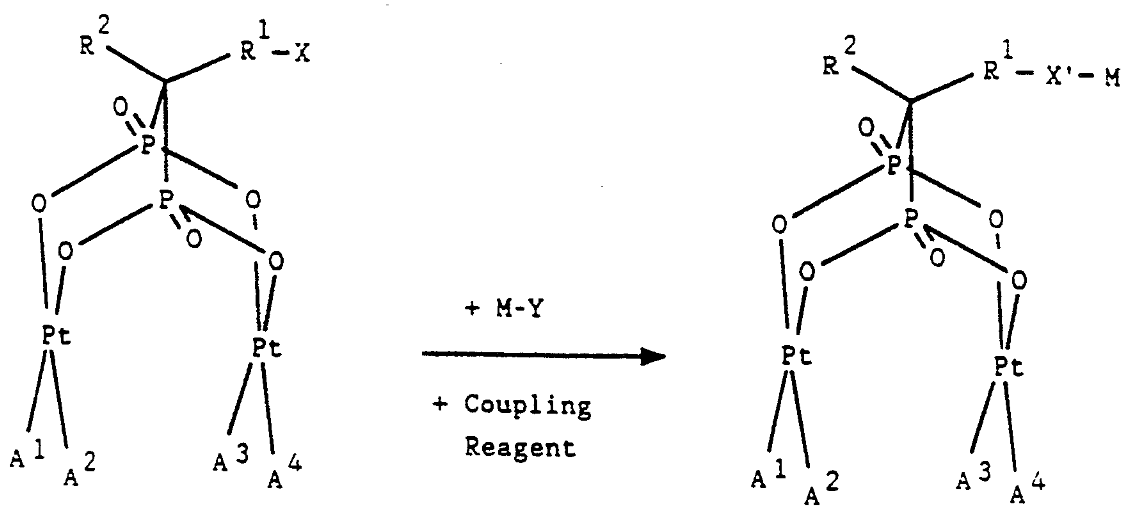


I

II

In this conjugation reaction, the molecule of interest (M) is attached to the Pt-DP complex (I) by reacting a suitable linker functionality (Y) on M with the reactive X group of the platinum complex. The coupling reaction, which may be assisted by a suitable coupling reagent, such as a carbodiimide, produces a bond (X′) between M and the Pt-DP complex, yielding the Pt-DP-conjugate (II).

For example, the Y functional group can be a selectively modified sugar residue, such as an aldehyde, attached to an antibody (M). In this case, the functional group (X) on the platinum complex could be an acid hydrazide or an amine which upon reaction would produce, respectively, a hydrazone or a Schiff's base linkage (X′) to the antibody. Examples of this type of conjugation of agents to antibodies can be found in U.S. Patent 4,741,900, which is incorporated herein by reference.

The platinum-diphosphonate complexes (I) can be attached to a variety of molecules (M), such as antibodies, hormones, growth factors, glycolipids, oligosaccharides, amino acids, peptides, organic dyes and DNA intercalation reagents, steroids, and cytotoxic drugs (such as existing antitumor agents), via the linker groups (X′) listed below. The various X′ linker groups are formed by using standard coupling reactions between the X and Y functional groups on the two reactants (Reaction 1).

Examples of some of the linkages that may be formed are shown below together with the identification of X′ for the particular linkage:

1) Ester linkage

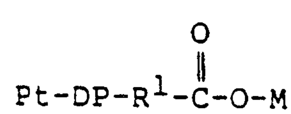

or

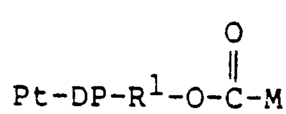

X' = 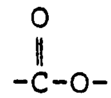

2) Amide linkage

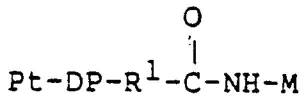

or

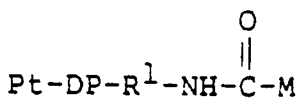

X' = 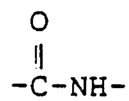

3) Schiff's base linkage, or reduced form thereof-

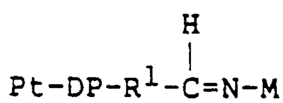

or

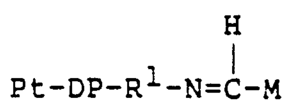

X' = 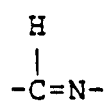

4) Ether linkage

$Pt-DP-R^1-O-M$

$X' = -O-$

5) Hydrazone, phenylhdrazone, or a semicarbazone linkage, or reduced form thereof-

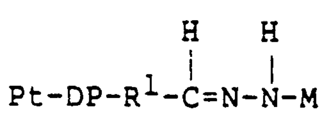

or

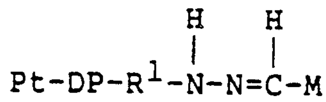

X' = 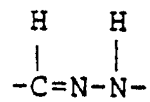

6) Aldehyde-derived hydrazone Linkage, or reduced forms thereof-

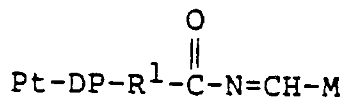

or

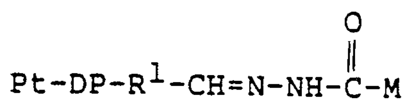

X' = 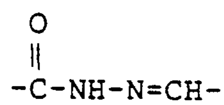

7) Alkyl amine linkage- wherein $R_4$ and $R_5$ are the same or different and are alkyl ($C_1$-$C_4$) or H.

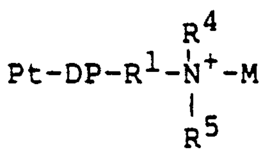

$X^1$ = 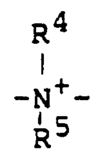

The following examples show how the Pt-DP complexes can be coupled to aminosugars and amino acid esters using a carbodiimide coupling reagent (EDCI, 1-(3-dimethylaminopropyl)-3-ethylcarbodiimide-HCl).

REACTION 2

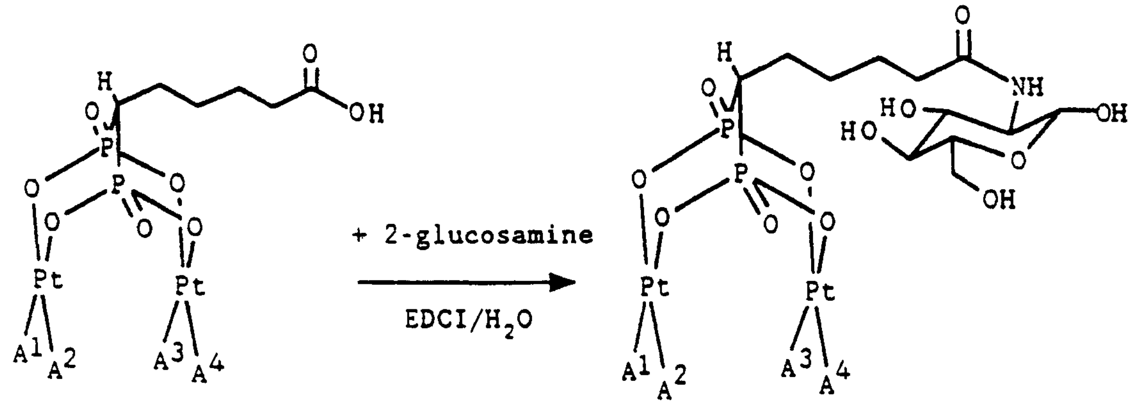


III IV

REACTION 3

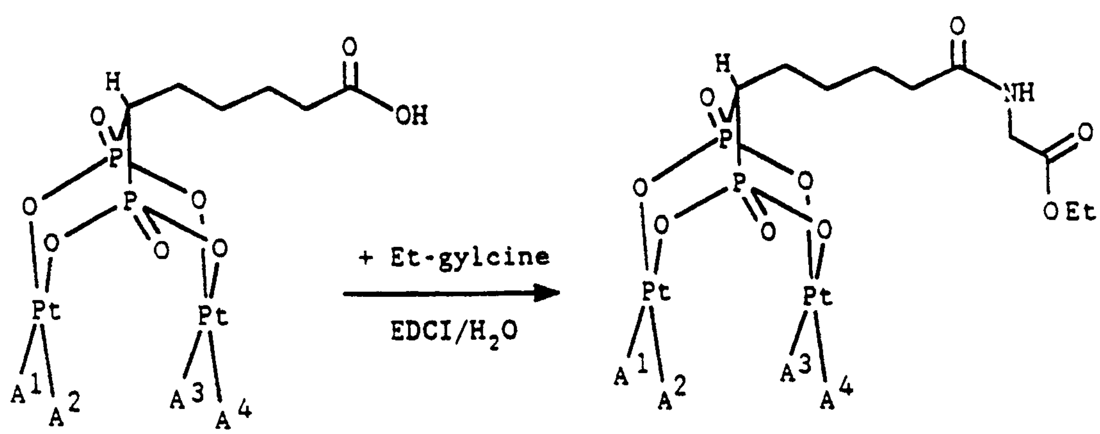


III V

A second example of conjugate formation between the Pt-PD complexes and the chosen molecule (M) is shown in reactions 4 and 5. This method involves the coupling of an aldehyde group (Y = -C(O)H) on the molecule to be coupled, with the acid hydrazide derivative (X = $-C(O)N_2H_3$) of the Pt-PD complex, to give a hydrazone linkage (X′ = -C(O)NHN = CH-) in the coupled product.

REACTION 4

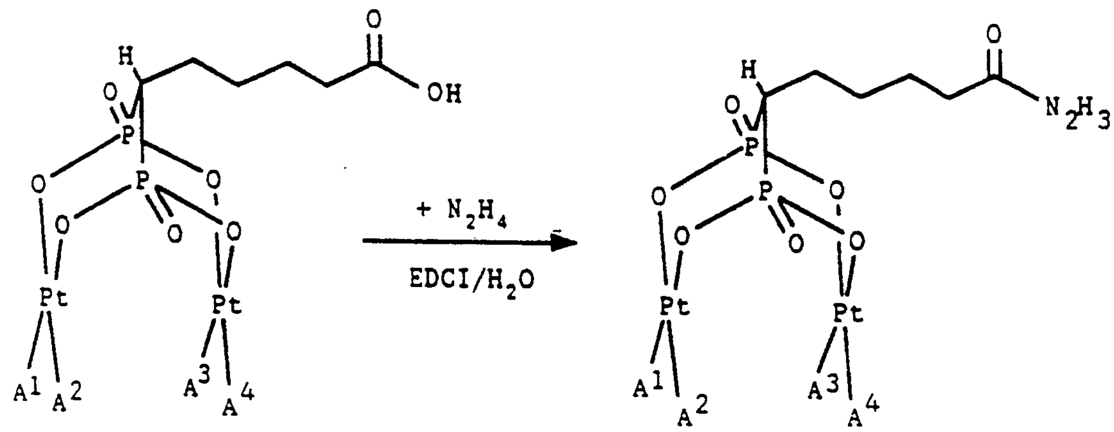


III VI

REACTION 5

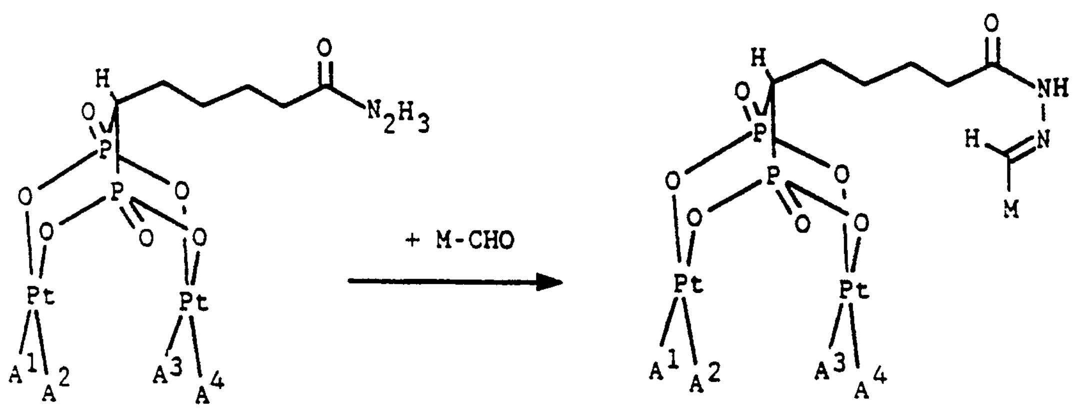


VI VII

In reaction sequences 2 and 3, the ethylenediamine Pt-DP complex, $Na[Pt_2(en)_2DP]$ (III), was used to study the coupling reactions of two model compounds, an amino sugar (D-glucosamine, Reaction 2, Example 5) and an amino acid ester (ethylgylcinate, Reaction 3, Example 6). Both reactions, which may be used to modify a variety of amine containing molecules of interest, were successfully carried out using the water-soluble carbodiimide coupling reagent EDCI. The coupling reaction in both cases was found to produce the desired products after 24 hours at room temperature in water. The coupled products (IV and V) were purified by ion exchange chromatography and were characterized by $^{195}Pt$, $^{31}P$, and $^{13}C$ NMR spectroscopy (see Tables 1-2).

By using this method, simple proteins can be coupled to the Pt-PD complexes, and by choosing receptor specific peptides, the complexes may be delivered in a site specific fashion. This technique also can be used to attach the Pt-DP complexes to know antitumor agents that contain amine functional groups, such as doxorubicin. The resulting combination could provide an agent with unique antitumor properties.

The acid hydrazide complex (VI) (reactions 4 and 5) is readily prepared by reacting the carboxylate group of the Pt-DP complex with three equivalents of hydrazine in the presence of EDCI in water. The resulting complex (VI) can be separated from hydrazine and EDCI impurities by precipitation from methanol. The hydrazide complex, which is stable in aqueous solutions, reacts with aldehydes to give the semicarbazone derivative (VII) (Bodanszky, M.; Bodanszky, A., in "The Practice of Peptide Synthesis", Springer-Verlag, New York, 1984). In this fashion, the Pt-DP complexes can be linked to suitably modified antibodies for the purpose of targeted drug delivery.

The products of this invention are useful in the treatment of malignant tumor cells sensitive thereto in animals as, for example, Sarcoma 180 ascites and L1210 leukemia in mammals, such as mice, either alone or as a mixture. This antitumor effect also may extend to other sarcomas and leukemias and to such other tumor cells as lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonal carcinoma, cystadenocarcinoma, endometroidcarcinoma or neuroblastoma and the like. In addition, the complexes may be useful as anti-viral, anti-inflammatory, anti-bacterial and antiparasitic agents.

The Pt-DP complexes of this invention may be administered parenterally or orally, alone or in a mixture, with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous, or intraarterial injection. In the case of the conjugates, they may be administered parenterally.

## EXAMPLES

### Example 1

Preparation of Hexane-1-carboxy-6,6-diphosphonic acid ($H_5PDP$)

Tetraethylmethanediphosphonate (75 mL) was added, over a period of 20 min, to a mixture of anhydrous THF (500 mL) and potassium metal (11.98 g). Ethyl-5-bromovalerate (52 mL) was added dropwise to the resulting solution over a period of 25 min. After refluxing for 2 hours, the mixture was stirred at room temperature for 12 hours and the resulting KBr precipitate was removed by filtration. The solvent was then removed by evaporation and the product was isolated by vacuum distillation (distilled 2 times, bp = 217 °C, at 1 mm Hg). The resulting ester (35 mL) was heated with stirring in an acqueous solution (2 X 75 mL) of HBr (47 wt%) for 4 hours. The HBr solution was then removed under vacuum and the resulting brown oil was dissolved in 250 mL of acetone (with 0.1 g Norit) and filtered. After removing the acetone by evaporation, the hydrolyzed product was dissolved in 100 mL of water and the pH of this solution was adjusted to 11.0 with 6 M NaOH. The adjusted solution was filtered and evaporated to dryness (yield 28 g). The product pentasodium hexane-1-carboxy-6,6-diphosphonate ($Na_5PDP$), was further purified by gel filtration (using Spectragel GF-05). The free acid form of the ligand, $H_5PDP$, can be obtained by acidifying (pH < 1) the sodium salt with 6-12 M HCL and evaporating the resulting solution under vacuum. The free acid can be separated from the resulting NaCl by extracting the mixture with acetone, filtering to remove the salt, and evaporating the filtrate under vacuum. The ligand was characterized by using NMR spectroscopy (see Tables 1 an 2).

### Example 2

Preparation of $Na[Pt_2(en)_2(PDP)]$

A suspension of 12.637 g of $[Pt(en)I_2]$ in 625 mL of water was treated with 8.44 g of $AgNO_3$ and the remaining mixture was heated to 55 °C for 3 h with stirring. After cooling to room temperature and removing the AgI by filtration, the filtrate was treated with 0.5 equiv of $Na_5PDP$. This solution was then heated to 50 °C for 3 h and the water was removed by evaporation (note: the $H_5PDP$ can also be used, and in this case the pH of the solution is adjusted to 6 with 6 M NaOH before heating). The resulting solid (12 g) was stirred in 12 mL of water and the pH was adjusted to 8.5 with 6 M NaOH. The solution was then filtered and the filtrate was treated with conc HCl (to pH = 1.5). The resulting precipitate was collected, after cooling for 1 hour at 5 °C, and washed with water, ethanol and ether (yield 5.3 g, after vacuum drying). The sodium salt can be obtained by dissolving the complex in water (adjusted to pH = 7, with 6 M NaOH) and precipitating the product with acetone. The complex was characterized by using NMR spectroscopy (see Tables 1 and 2).

Example 3

Preparation of $Na[Pt_2(R,R\text{-dach})_2(PDP)]$

A mixture of 9.791 g of $[Pt(R,R\text{-dach})I_2]$ in 300 mL of water was treated with 5.908 g of $AgNO_3$ heated to 55 °C for 3 h. After cooling to room temperature, the AgI was removed by filtration, and 0.5 equiv of $Na_5PDP$ (in 20 mL water) was added to the filtrate. The resulting solution was stirred at room temperature for 18 h and a grey precipitate was isolated by filtration. This material was suspended in 150 mL of water and the pH adjusted to 7.2 with 2 M NaOH. After filtering the solution to remove insoluble materials, the filtrate was evaporated to dryness and the resulting product was dried under vacuum. Additional purification was accomplished by dissolving a portion of this sample (1.3 g) in 30 mL of water (trace ascorbic acid was used to discharge the green color), filtering and readjusting the pH to 2 with 1 M $HNO_3$. The resulting precipitate, $H[Pt_2(R,R\text{-dach})_2(PDP)]$, was collected by filtration and washed with water and acetone (yield 0.9 g after vacuum drying). The product was characterized by using NMR spectroscopy (see Tables 1 and 2) and elemental analysis. Anal. Calc'd. for $Pt_2C_{18}H_{49}N_4P_2O_{14}$ Na: C, 21.18; H, 4.84; N, 5.49; P, 6.07; Na, 2.25. Found: C, 21.17; H, 4.71; N, 5.55; P, 5.28; Na, 2.29.

Example 4

Preparation of $[Pt_2(R,R\text{-dach})_2(HEDP)]$

Silver nitrate (1.21 g) was added to a suspension of 2 g of $[Pt(R,R\text{-dach})I_2]$ in 10 mL of water. After stirring for 3 h at 55 °C, the mixture was cooled to room temperature and the AgI was removed by filtration. The filtrate was treated with 0.5 equiv of 1-hydroxyethane-1,1-diphosphonate (HEDP) and the pH was adjusted to 7 with 1 M NaOH. After heating to 45 °C for 2 h, the solution was cooled to 25 °C and poured into 200 mL of acetone. The resulting precipitate was isolated by filtration and dried under vacuum (yield 1.28 g). A portion of this material was dissolved in water (0.65 g in 10 mL) and passed over a mixed bed cation exchange resin (Amberlite MB-1) to remove starting materials. The eluant was collected and evaporated to dryness, yielding 200 mg of product after vacuum drying. The complex was characterized by using NMR spectroscopy (see Tables 1 and 2).

Example 5

Preparation of $[Pt_2(en)_2(PDP\text{-D-glucosamine})]$

A sample of $H[Pt_2(en)_2(PDP)]$ (1.266 g) was placed in 5 mL of water with 0.25 g of D-glucosamine.HCl, and the pH as adjusted to 10 with NaOH. The coupling reagent 1-(3-dimethylamineopropyl)-3-ethylcarbodiimide.HCl (EDCI, 0.311 g) was added to this solution (resulting pH = 6.7) and the reaction was allowed to proceed for 14 h at 25 °C. An additional 50 mg of EDCI was then added, and after 1 h the solution was passed over a mixed bed cation exchange resin (Amberlite MB-1). The eluant was evaporated to dryness, redissolved in water (3 mL) and precipitated with acetone (yield 600 mg). The product was characterized by $^{195}Pt$, $^{31}P$ and $^{13}C$ NMR spectroscopy (see Tables 1 and 2).

Example 6

Preparation of $[Pt_2(en)_2(PDP\text{-ethylglycinate})]$

$Na[Pt_2(en)_2(PDP)]$ (0.783 g) and ethylglycinate.HCl (0.140 g) were placed in 3 mL of water with 0.192 g of EDCI and the pH of the solution was adjusted to 7 with 6 M NaOH. After 20 h at 25 °C, the solution was passed over a mixed bed ion exchange resin (Amberlite MB-1) and the eluant was evaporated to a volume of 3 mL. The product was precipitated with acetone and dried under vacuum, and characterized by using

NMR spectroscopy (see Tables 1 and 2).

Example 7

Preparation of $[Pt_2(en)_2(PDP\text{-hydrazide})]$

$H[Pt_2(en)_2(PDP)]$ (1.156 g) was placed in 3 mL of water and the pH was adjusted to 8 with 6 M NaOH. EDCI (0.576 g) was added to the solution along with 0.174 g of anhydrous hydrazine, and the pH was readjusted to 6.8 with 2 M $HNO_3$. After 2 h, the solution was filtered to remove insoluble materials and the filtrate was added to 75 mL of methanol with stirring. The resulting precipitate was filtered, washed with methanol and vacuum dried (yield 800 mg). The complex was characterized by using NMR spectroscopy (see Tables 1 and 2).

Compound Characterization

All compounds were characterized by using $^{31}P$, $^{195}Pt$ and $^{13}C$ NMR spectroscopy. A listing of chemical shifts for each analog and relevant starting materials is given in Tables 1 and 2. Elemental analysis were obtained on one analog (Galbraith Laboratories, Knoxville, TN), and this data is reported in the compound preparation section.

Table 1.

$^{31}P$ and $^{195}Pt$ NMR Data for the Platinum-Diphosphonate Complexes.

| Complex/Ligand[a] | $^{31}P$ (ppm)[b] | $^{195}Pt$ (ppm)[c] |
|---|---|---|
| $[Pt_2(R,R\text{-dach})_2(HEDP)]$ | 43.4(q) | -1659 |
| $Na[Pt_2(R,R\text{-dach})_2(PDP)]$ | 44.5(q) | -1657 |
| $[Pt_2(en)_2(PDP\text{-}N_2H_3)]$ | 45.1 | -1698 |
| $Na[Pt_2(en)_2(PDP)]$ | 44.4 | -1715 |
| $[Pt_2(en)_2(PDP\text{-glucosamine})]$ | 44.0 | -1701 |
| $[Pt_2(en)_2(PDP\text{-Et-glycine})]$ | 44.2 | -1708 |
| $PDP(OEt)_5$ | 19.6 | |
| $MDP(OEt)_4$ | 18.7 | |
| $H_4HEDP$ | 22.1 | |
| $H_5PDP$ | 24.4 | |

[a] Abbreviations: HEDP, 1-hydroxyethane-1,1-diphosphonate; MDP, methane-1,1-diphosphonate; PDP, hexane-1-carboxy-6,6-diphosphonic acid; $PDP\text{-}N_2H_3$, acid hydrazide derivative of PDP, q = quartet

[b] Ref. to $H_3PO_4$ at 0 ppm.

[c] Ref. to $H_2PtCl_6$ at 0 ppm.

Table 2. $^{13}C$ NMR Data for the Platinum-Diphosphonate Complexes.

| Compound | -Diamine Ligand- | | | -DP Ligand- | | | | | | -Linked Group- | | | | | |
|---|---|---|---|---|---|---|---|---|---|---|---|---|---|---|---|
| | C1',2' | C3',5' | C4',6' | C1 | C2 | C3 | C4 | C5 | C6 | C1 | C2 | C3 | C4 | C5 | C6 |
| $[Pt_2(R,R\text{-dach})_2(HEDP)]$[b] | 65.73<br>63.24<br>63.15<br>63.16 | 32.53<br>32.29 | 24.71<br>24.50 | 75.62<br>J=207.6 | 19.18 | | | | | | | | | | |
| $Na[Pt_2(R,R\text{-dach})_2(PDP)]$[a] | 65.69<br>65.64<br>63.25<br>63.14 | 32.62<br>32.37 | 24.64<br>24.56 | 44.20<br>J=175.7 | 29.86 | 26.44 | 26.81 | 38.05 | 184.59 | | | | | | |
| $Na[Pt_2(en)_2(PDP)]$[a] | 49.43 | | | 44.03<br>J=176.2 | 29.64 | 26.25 | 26.53 | 37.78 | 183.87 | | | | | | |
| $[Pt_2(en)_2(PDP\text{-hydrazide})]$[b] | 50.57 | | | 44.79<br>J=175.3 | 29.73 | 26.85 | 26.51 | 34.69 | 176.84 | | | | | | |
| $[Pt_2(en)_2(PDP\text{-Et-gylcine})]$[b] | 49.12 | | | 43.61<br>J=175.9 | 28.65 | 25.70 | 25.29 | 35.02 | 177.43 | 62.24 | 171.7 | 41.26 | 13.21 | | |
| $[Pt_2(en)_2(PDP\text{-glucosamine})]$[b] | 49.15 | | | 43.81<br>J=175.2 | 28.38 | 25.76 | 25.62 | 35.44 | 177.68<br>177.53 | 94.96<br>90.82 | 56.76<br>54.17 | 73.74<br>70.48 | 70.15<br>69.95 | 75.98<br>71.68 | 60.83<br>60.68 |
| $H_4HEDP$[c] | | | | 70.73<br>J=139.2 | 19.22 | | | | | | | | | | |
| $H_5PDP$[c] | | | | 36.48<br>J=207.0 | 27.33 | 24.02 | 23.35 | 32.79 | 178.04 | | | | | | |
| Et-gylcinate[b] | | | | | | | | | | 63.38 | 170.5 | 41.35 | 13.89 | | |
| D-glucosamine[b] | | | | | | | | | | 89.57<br>93.16 | 54.77<br>57.22 | 70.06<br>72.45 | 70.06<br>70.12 | 72.03<br>76.51 | 60.82<br>60.97 |

[a] Ref. to ethanol at 18.2 ppm. [b] Ref. to methanol at 49.4 ppm. [c] Ref. to acetone at 29.8 ppm.

## Compound Screening

The platinum-diphosphonate complexes were tested for antitumor activity using both Sarcoma 180 ascites and L1210 leukemia tumor models (see Tables 3 and 4). While each compound has not been tested in both screens and while certain compounds do not show activity in certain screens, certain compounds may show activity in other screens.

## L1210 Screening methodology

Female $CDF_1$ mice (16-22g) were injected with a suspension of $1x10^6$ tumor cells in 0.5 mL of 0.9 M saline on day zero. On day one, the mice received the test compound in 0.5 mL of the chosen medium (usually water). Six mice were used for each test dose and six doses were normally given. Normal controls (six mice) received tumor on day zero and 0.5 mL of test medium on day one. Positive controls (six mice) were given tumor on day zero and 8 mg/kg cisplatin in 0.5 mL of 0.9 M saline on say one. All compound doses were adjusted for the actual average weight of the mice involved. Testing was terminated at 3 time the normal control mean life span. The percent increase in lifespan (%ILS) was calculated as follows:

%ILS = [(mean life span (test)/mean life span (control))-1] X 100%

A 25% ILS or greater indicates activity in this screen. The maximum possible ILS is 200%. The positive control must have an ILS > 25% for the test to be considered valid.

## S180a Screening Methodology

Female CFW mice (18-25g) were implanted with 2 x $10^6$ cells ip on day zero. Compounds were administered in 0.5 mL water ip on say one. Groups of 6 mice were used for each test dose as well as controls as defined above. The test was run for twice the MST of the control group, with survivors counted as dying on that day. An ILS of > 50% indicates activity. The tumor line was maintained through weekly transfer of 4 x $10^6$ cells in CFW mice.

The results of the screening tests are reported in Tables 3 and 4.

| 3. S180a Screening Data for the Platinum-Diphosphonate Complexes | | | | | |
|---|---|---|---|---|---|
| COMPOUND Example/Vehicle | DOSE mg/kg | %ILS | SURV | POS %ILS | CONT SURV |
| $[Pt_2(R,R\text{-}dach)_2(HEDP)]$ Example 4/water | 10.00<br>20.00<br>40.00<br>80.00<br>160.00<br>256.00 | -7<br>-10<br>10<br>4<br>77<br>47 | 0/ 6<br>0/ 6<br>0/ 6<br>0/ 6<br>3/ 6<br>1/ 6 | 82 | 3/ 6 |
| | 20.00<br>40.00<br>80.00<br>160.00<br>320.00 | -4<br>-3<br>16<br>95<br>75 | 0/ 6<br>0/ 6<br>0/ 6<br>5/ 6<br>4/ 6 | 63 | 1/ 6 |
| $Na[Pt_2(en)_2(PDP)]$ Example 2/water (pH 7) | 10.00<br>20.00<br>40.00<br>80.00<br>160.00<br>320.00 | -4<br>5<br>6<br>27<br>48<br>49 | 0/ 6<br>0/ 6<br>0/ 6<br>1/ 6<br>1/ 6<br>1/ 6 | 58 | 2/ 5 |
| $[Pt_2(en)_2(PDP\text{-}glucosamine)]$ Example 5/water | 10.00<br>20.00<br>40.00<br>80.00<br>160.00<br>320.00 | 0<br>-3<br>-5<br>0<br>0<br>28 | 0/ 6<br>0/ 6<br>0/ 6<br>0/ 6<br>0/ 6<br>0/ 5 | 92 | 4/ 6 |

| 4. L1210 Screening Data for the Platinum-Diphosphonate Complexes | | | | | |
|---|---|---|---|---|---|
| COMPOUND Example/Vehicle | DOSE mg/kg | %ILS | SURV | POS %ILS | CONT SURV |
| $[Pt_2(R,R\text{-dach})_2(HEDP)]$ Example 4/water | 10.00<br>20.00<br>40.00<br>80.00<br>160.00<br>320.00 | 8<br>3<br>24<br>28<br>40<br>72 | 0/ 6<br>0/ 6<br>0/ 6<br>0/ 6<br>0/ 6<br>0/ 6 | 70 | 0/ 6 |
| $Na[Pt_2(R,R\text{-dach})_2(PDP)]$ Example 3/water | 10.00<br>20.00<br>40.00<br>80.00<br>160.00<br>320.00 | 1<br>28<br>28<br>55<br>104<br>111 | 0/ 6<br>0/ 6<br>0/ 6<br>0/ 6<br>2/ 6<br>2/ 6 | 106 | 1/ 6 |
| $[Pt_2(en)_2(PDP\text{-glucosamine})]$ Example 5/water | 10.00<br>20.00<br>40.00<br>80.00<br>160.00<br>320.00 | -8<br>-15<br>10<br>4<br>13<br>16 | 0/<br>0/ 6<br>0/ 6<br>0/ 6<br>0/ 6<br>0/ 6 | 117 | 3/ 6 |
| $Na[Pt_2(en)_2(PDP)]$ Example 2/water | 10.00<br>20.00<br>40.00<br>80.00<br>160.00<br>320.00 | 0<br>-3<br>0<br>20<br>23<br>11 | 0/ 6<br>0/ 6<br>0/ 6<br>0/ 6<br>0/ 6<br>0/ 6 | 166 | 4/ 6 |
| $[Pt_2(en)_2(PDP\text{-}N_2H_3)]$ Example 7/water | 10.00<br>20.00<br>40.00<br>80.00<br>160.00<br>320.00 | -14<br>21<br>-18<br>-14<br>20<br>0 | 0/ 6<br>0/ 6<br>0/ 6<br>0/ 6<br>0/ 6<br>0/ 6 | 41 | 0/ 6 |

## Claims

1. A compound of the general formula I

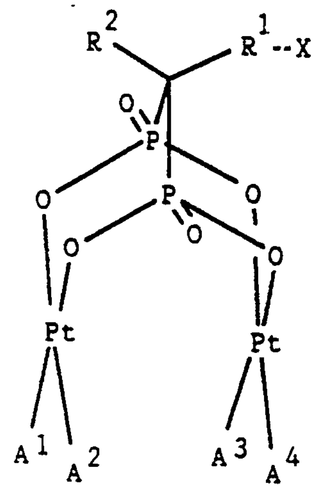

I

wherein the A ligands are the same or different and when taken alone are selected from a monodentate amine, a primary alkylamine having the formula $R^3NH_2$, or a secondary alkylamine having the formula $R^3{}_2NH$, wherein $R^3$ is selected from $C_1$-$C_6$ linear, cyclic or branched; wherein, when $A^1$ and $A^2$, and $A^3$ and $A^4$ are taken together they each form a bidentate diamine or a $C_4$ to $C_8$ cycloalkyl diamine, wherein $R^1$ and $R^2$ are the same or different and are selected from H, substituted or unsubstituted $C_1$-$C_6$ alkoxy, substituted or unsubstituted $C_1$-$C_6$ linear or branched alkyl, substituted or unsubstituted $C_1$-$C_6$ linear or branched hydroxyalkyl, substituted or unsubstituted $C_1$-$C_6$ linear or branched alkyl ether, substituted or unsubstituted $C_4$-$C_8$ cycloalkyl, substituted or unsubstituted phenyl, or the functional group X, wherein the substituents and X are the same and are selected from amine, hydroxyl, halo, carboxylic acid, carboxylic acid halide, carboxylic acid anhydride, carboxylic acid hydrazide, ester, amide, aldehyde, acetal, hemicetal, ketone, ketal, -O-acylurea, sulfonic acid ester, hydrazine, substituted or unsubstituted $C_1$-$C_6$ linear or branched alkenyl, or a substituted or unsubstituted phenyl; with the provision that when $A^1$ and $A^2$, and $A^3$ and $A^4$ are ethylenediamine $R^1$ and $R^2$ cannot both be H, or cannot be $CH_3$ and OH, respectively, and when $A^1$ and $A^2$, and $A^3$ and $A^4$ are cis-diaminocyclohexane, $R^1$ and $R^2$ cannot both be H.

2. The compound of Claim 1, wherein $A^1$ and $A^2$, and $A^3$ and $A^4$ when taken together each form ethylenediamine.

3. The compound of Claim 1, wherein $A^1$ and $A^2$, and $A^3$ and $A^4$ are taken together each form 1,2-diaminocyclohexane.

4. The compound of Claim 1, wherein $R^1$ and $R^2$ are selected from H, OH, or $CH_3$.

5. The compound of Claim 1, wherein $R^1$ is $(CH_2)_5$, $R^2$ is H, X is $CO_2^-$ and $A^1$ and $A^2$, and $A^3$ and $A^4$, taken together, each form ethylenediamine.

6. The compound of Claim 1, wherein $R^1$ is $(CH_2)_5$, $R^2$ is H, X is $CO_2^-$ and $A^1$ and $A^2$, and $A^3$ and $A^4$ when taken together, each form 1,2-diaminocyclohexane.

7. The compounds of Claim 1, wherein $R^1$ is X and X is OH, $R^2$ is $CH_3$, $A^1$ and $A^2$, and $A^3$ and $A^4$, when taken together each form 1,2-diaminocyclohexane.

8. A composition for treating malignant animal tumor cells sensitive thereto, said composition comprising as active ingredient a compound according to any of claims 1 to 7, in admixture with a pharmacologically acceptable inert carrier or diluent, wherein the active ingredient is present in an amount sufficient to cause regression of tumors sensitive thereto.

9. A composition according to claim 8, which is in the form of a tablet.

10. A composition according to claim 8, which is in the form of a liquid.

11. A method of treating malignant animal tumors sensitive to a compound according to any of claims 1 to 7, comprising administering the compound or a mixture of such compounds to an animal afflicted with such a tumor in an amount sufficient to cause regression of the tumor.

12. A conjugate of the general formula II:

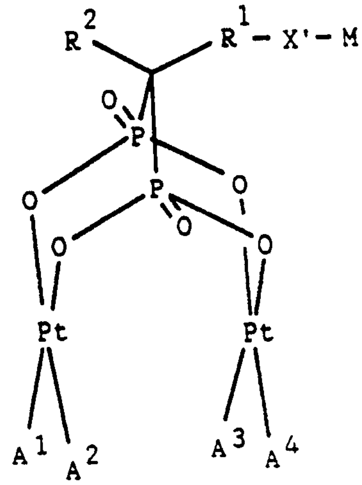

II

wherein the A ligands are the same or different and when taken alone are selected from a monodentate amine, a primary alkylamine having the formula $R^3NH_2$, or a secondary alkylamine having the formula $R^3{}_2NH$, wherein $R^3$ is selected from $C_1$-$C_6$ linear, cyclic or branched; wherein when $A^1$ and $A^2$, and $A^3$ and $A^4$ are taken together they each form a bidentate diamine or a $C_4$ to $C_8$ cycloalkyl diamine, wherein $R^1$ and $R^2$ are the same or different and are selected from H, substituted or unsubstituted $C_1$-$C_6$ alkoxy, substituted or unsubstituted $C_1$-$C_6$ linear or branched alkyl, substituted or unsubstituted $C_1$-$C_6$ linear or branched hydroxyalkyl, substituted or unsubstituted $C_1$-$C_6$ linear or branched alkyl ether, substituted or unsubstituted $C_4$-$C_8$ cycloalkyl, substituted or unsubstituted phenyl, wherein X′ is selected from

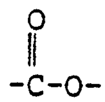 ,  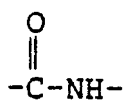 ,  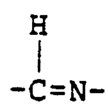 ,

-O-,  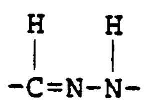 ,  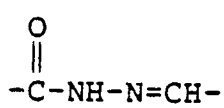  or  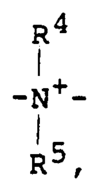 ,

>

wherein $R^4$ and $R^5$ are the same or different and selected from $C_1$-$C_4$ alkyl or H; wherein M is a target molecule.

13. The conjugate of Claim 12 , wherein M is selected from antibodies, hormones, growth factors, glycosides, oligosaccharides, amino acids, peptides, organic dyes, DNA intercalation reagents, steroids or cytotoxic drugs.

14. The compound of Claim 12 , wherein M is glucose, X′ is

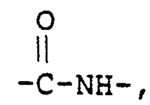

$A^1$ and $A^2$, and $A^3$ and $A^4$ when taken together, each form ethylenediamine, wherein $R^2$ is H and wherein $R^1$ is $(CH_2)_5$.

15. The conjugate of Claim 12 , wherein $R^1$ is $(CH_2)_5$, $R^2$ is H, $X'$ is

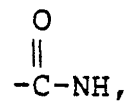

$A^1$ and $A^2$, and $A^3$ and $A^4$ when, taken together, each form ethylenediamine, and M is

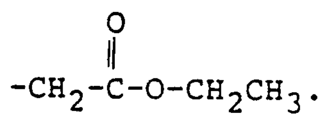

16. A composition for treating malignant animal tumor cells sensitive thereto, said composition comprising as active ingredient a compound according to any of claims 12 to 15, in admixture with a pharmacologically acceptable inert diluent or carrier.

17. A method of treating malignant animal tumors sensitive to a compound according to any of claims 12 to 15, comprising administering the compound or a mixture of such compounds to an animal afflicted with such a tumor in an amount sufficient to cause regression of the tumor.

18. A method of making a composition for the treatment of malignant animal tumor cells, which composition comprises as active ingredient a compound according to any of claims 1 to 7 and 12 to 15, comprising mixing the said active ingredient with a pharmacologically acceptable diluent or carrier.

19. A compound according to any of claims 1 to 7 and 12 to 15, for use in the treatment of the human or animal body by therapy.

20. The use of a compound according to any of claims 1 to 7 and 12 to 15 in the manufacture of a medicament for treating animal malignant tumor cells.